Europäisches Patentamt

European Patent Office

Office européen des brevets

⑩

(11) Publication number: **0 263 489**
**B1**

(12) **EUROPEAN PATENT SPECIFICATION**

(45) Date of publication of patent specification: **23.01.91**

(21) Application number: **87114583.5**

(22) Date of filing: **06.10.87**

(51) Int. Cl.⁵: **C 04 B 35/00,** C 04 B 38/06, A 61 L 27/00, C 12 N 11/14, B 01 J 32/00

(54) **Granular inorganic moldings and a process for production thereof.**

(30) Priority: **06.10.86 JP 238947/86**

(43) Date of publication of application:
**13.04.88 Bulletin 88/15**

(45) Publication of the grant of the patent:
**23.01.91 Bulletin 91/04**

(84) Designated Contracting States:
**DE FR GB IT NL**

(56) References cited:
**EP-A-0 054 333**
**EP-A-0 177 727**
**JP-A-61 020 558**
**US-A-2 847 710**

(73) Proprietor: **KURARAY CO., LTD.**
**1621 Sakazu**
**Kurashiki-City Okayama Prefecture 710 (JP)**

(72) Inventor: **Nakamura, Seishiro**
**1000-107, Hashima**
**Kurashiki-city, 710 (JP)**
Inventor: **Oukami, Katutoshi**
**635-8, Misu**
**Soja-city, 719-11 (JP)**
Inventor: **Asada, Masayuki**
**858-12, Mizue**
**Kurashiki-city, 710 (JP)**

(74) Representative: **Vossius & Partner**
**Siebertstrasse 4 P.O. Box 86 07 67**
**D-8000 München 86 (DE)**

Courier Press, Leamington Spa, England.

**Description**

The present invention relates to granular inorganic moldings comprising inorganic compounds such as calcium phosphates, metal oxides and a mixture thereof, and a process for production thereof. The granular inorganic moldings are useful as fillers for bone defects or as carriers for immobilized enzymes or catalysts.

Granular calcium phosphate moldings which are representative of the granular inorganic moldings have properties of promoting formation of new bone and integrating with the living bone tissue and have thus been attempted to utilize as bone fillers.

As processes for production of granular calcium phosphate moldings, there are known (a) a process which comprises grinding the dry product of calcium phosphate prepared by the wet process into granules as it is or after precalcination, and then sieving the granules (Journal of American Chemical Society, 89, 5535 (1967), Published Unexamined Japanese Patent Application No. 20558/86, etc.), (b) a process which comprises repeatedly stirring calcium phosphate powder with a high speed stirring machine in the presence of alcohol as a medium while moisturizing to make granules having a desired size from the powder (Published Unexamined Japanese Patent Application No. 45748/87). The granular calcium phosphate moldings obtained by the processes as described above have an uneven shape of the granules so that, in the case of using them as bone fillers, problems are often encountered in that the filling state is not uniform and they do not exhibit stable efficiency. In addition, the granular calcium phosphate moldings obtained by the process (a) described above have some edges so that the edges stimulate bone cells and there is a fear of causing necrosis of the cells, in the case of filling them in bone defects. Further the granular calcium phosphate moldings obtained by the process (b) described above give high density granules only with difficulty, even after calcination and there is also a problem that mechanical strength is poor for the use of bone fillers.

Furthermore, in case that granules having a desired size are attempted to be produced by these processes, there is a problem that yield is poor due to production of powder in large quantities in any of the processes (a) and (b).

Accordingly, an object of the present invention is to provide granular inorganic moldings, especially calcium phosphate moldings having a uniform shape and which are useful as a bone filler.

Another object of the present invention is to prepare such granular inorganic moldings in a high yield.

These objects can be achieved by granular inorganic moldings which are characterized in that the ratio A/D of the maximum diameter D and the maximum length A perpendicular to the maximum diameter of the granular molding is 0.5 to 0.9 and in that at least 80% of the granular moldings have a shape such that the crossing point of A and D is within 0.3 D from both sides of the center of D. These granular inorganic moldings can be obtained in a high yield by filling an inorganic powder capable of calcination into an organic porous body having a 3-dimensional net-like structure, and subjecting it to press forming thereby to form granules of the inorganic powder in the pores of the porous body and then burning off (calcining) the organic porous body and at the same time, calcining the granular inorganic moldings.

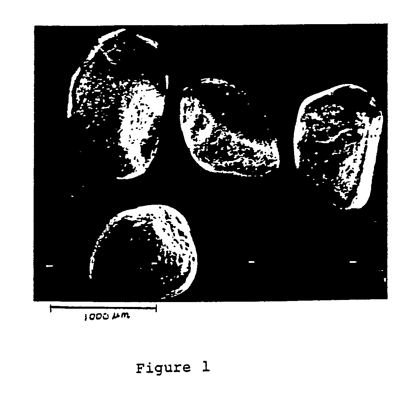

Figure 1 is an electron microscope photograph showing an example of granular calcium phosphate moldings obtained by the present invention.

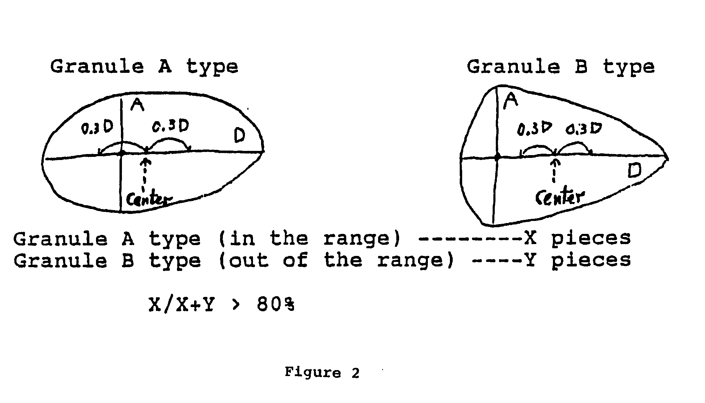

Figure 2 shows a granular molding in which the crossing point of A and D is within 0.3 D from both sides of the center of D (type A) and another granular molding in which the crossing point of A and D is outside of said range (type B). At least 80% of the granular moldings of the invention are type A granules.

As inorganic components constituting the granular inorganic moldings of the present invention, mention may be made of those mainly composed of calcium phosphates and those mainly composed of metal oxides as described below.

(1) Calcium phosphates such as tricalcium phosphate, hydroxyl apatite, tetracalcium phosphate, oxy apatite, calcium pyrophosphate, fluoro apatite, compounds in which a part of hydroxyl apatite is substituted with fluorine ions, and a mixture thereof, especially tricalcium phosphate, hydroxyl apatite and a mixture thereof are preferred. Other inorganic components, for example, metal oxides such as alumina and zirconia may be mixed with calcium phosphate in a small quantity (about 20% or less by weight based on calcium phosphate).

(2) Metal oxides such as alumina, zirconia, titania, silica, silica alumina or a mixture thereof.

The granular inorganic moldings of the present invention is characterized in that the ratio A/D (when the maximum diameter is made D and the maximum length perpendicular to the maximum diameter is made A) is 0.5 to 0.9 and in that at least 80% of the granular moldings have a shape such that the crossing point of A and D is within 0.3 D from both sides of the center of D.

Therefore, the shape of each granule constituting a group of granules is uniform so that always stable efficiency can be obtained. Measurement of such a shape of the granules can be performed on an electron microscope photograph.

If A/D is less than 0.5, the granules are easily powdered owing to sharp edged shapes. On the other hand, if A/D is more than 0.9, it is difficult to fill densily the granules, and the filled granules are movile. Further, if the above-mentioned number of granules is less than 80%, it is difficult to obtain a uniform filling state.

As will be described below, the granular inorganic moldings of the present invention are prepared by

2

filling an inorganic powder capable of calcination into an organic porous body having a 3-dimensional net-like structure and subjecting it to press forming thereby to form granules of the inorganic powder in the pores of the porous body and then burning off the organic porous body. Therefore, the granular inorganic moldings of the present invention are characterized in that each granule is not only uniform in its shape but also free of edges. The granular inorganic moldings of the present invention are also characterized in that they have a high density depending upon the applied pressure and the calculation temperature. They are polyhedral depending upon the shape of the pores of the organic porous body used. Linear grooves may be observed on the surface of the granules.

The granular inorganic moldings of the present invention can be obtained by press forming of a powder of the inorganic compounds, mainly composed of calcium phosphates or metal oxides as described above, in the pores of an organic porous body. The raw powder used in the present invention has generally a size of 0.5 to 500 μm with particular preference of 1 to 200 μm, which size is appropriately chosen depending upon the size of the granules to be obtained. It is also desired that the content of water in the powder is 5% or less.

An example of the organic porous body having a 3-dimensional net-like structure used in the present invention includes a polyurethane foam. The size and shape of the polyurethane foam can be controlled depending upon the conditions for preparation. Accordingly, the size and shape of the granular inorganic moldings can be varied by selecting the size and shape of the cells of the polyurethane foam. By the use of such a foam, granular inorganic moldings up to a maximum diameter of 0.1 to 2 mm which is the size of ordinary granules can be prepared. The porous body having a 3-dimensional net-like structure is not limited to the polyurethane foam but any organic porous body having cells of a size and shape similar to the polyurethane foam is usable; those made of polyethylene or polystyrene can also be used. It is preferred that the porosity be as large as possible; with a porosity of 80% or more, the desired shape can be obtained in a good yield. In order to prepare a group of granules having a uniform shape in a good yield, it is required that the diameter distribution of the pores is uniform. However, commercially available porous bodies having a 3-dimensional net-like structure have sufficient uniformity to prepare the granular inorganic moldings of the present invention.

The inorganic powder is packed in the organic porous body described above and then press formed.

Preferred is isotropic pressure molding, inter alia, cold isostatic press fabrication. In the cold isostatic press fabrication, the organic porous body is put in close contact with a rubber type mold (usually made of rubber but may be made of any elastic body other than rubber).

The rubber type mold containing the inorganic powder-filled porous body is pressed for example by cold isostatic press fabrication (also called rubber press method), with a pressure of generally about 300 to 6000 bar (300 to 6000 kg/cm$^2$). With a pressure of less than about 300 bar (300 kg/cm$^2$) it is difficult to obtain moldings having a sufficient strength. With a pressure of more than about 6000 bar (6000 kg/cm$^2$) cracks may occur in the granular inorganic moldings when they are subsequently calcined. By performing the press fabrication as described above, the inorganic powder becomes granular in the pores of the porous body.

After the press fabrication is performed, the organic porous body is burned out, whereby the granular inorganic moldings can be taken out. At the same time, the inorganic moldings are calcined to achieve high density and increase the mechanical strength.

In the case of calcium phosphate moldings, it is preferred that the heating is conducted at 500 to 1400°C, more preferably 600 to 1300°C. With lower than 500°C, carbon of the organic porous body remains in the calcium phosphate calcination products and this is not preferred. With higher than 1400°C, cracks tend to occur in the calcium phosphate calcination products obtained. In case that porous granular calcium phosphate calcination products are required, it is desired to calcine at temperatures of 900°C or less; in case that dense granules (relative density of 90% or more) are required, it is desired to calcine at temperatures of 900°C or more.

Further in the case of the metal oxide moldings, it is preferred that the heating be carried out at 500 to 2000°C. With lower than 500°C, carbon of the organic porous body remains in the metal oxide calcination products and this is not preferred. With higher than 2000°C, cracks tend to occur in the metal oxide calcination products obtained. In case that porous granular metal oxide calcination products are required, it is desired to calcine at temperatures of 1300°C or less; in case that dense granules (relative density of 90% or more) are required, it is desired to calcine at temperatures of 1300°C or more, for example, for alumina.

As above, the granular inorganic moldings having a uniform shape can be obtained in a high yield by filling the inorganic powders into an organic porous body having a 3-dimensional net-like structure, performing press fabrication and then burning off the organic porous body. The granules obtained by the process of the present invention not only have a uniform shape but also are free of edges. Further depending upon press conditions or calcination conditions, the granules have a high density of 90% or more in relative density and high mechanical strength. Furthermore, the granules have a polyhedral shape depending upon the shape of the pores of the organic porous body used and it is also a characteristic that linear grooves are observed on the surface of a part of the granules. In addition, the process of the present invention is advantageous in that the granules can be obtained in a high yield but their manufacture is simple.

Hereafter the present invention will be described in more detail with reference to the examples below but is not deemed to be limited thereto.

3

**Example 1**

Calcium phosphate powder (Ca/P atomic ratio=1.67; grain diameter of the powder: 5—20 μm; specific surface area: 59 m$^2$/g) manufactured by Taihei Chemical Industrial Co., Ltd. was packed in 10 pieces of polyurethane foam "Everlightscott Filter" (HR-13; cell number: 11—16 cells/25 mm; porosity: 97%) manufactured by Bridgestone Corporation inserted in a rubber form (inner volume of 2 cm×5 cm×5 cm) and pressed with a rubber press under a pressure of about 2000 bar (2000 kg/cm$^2$). Thereafter the polyurethane foam containing the calcium phosphate powder (the calcium phosphate powder was in granular form in the pores of the foam) was put in an electric furnace and calcined at 500°C for 3 hours. After elevating the temperature to 1200°C, calcination was performed for 2 hours. The thus obtained granular calcium phosphate calcination product was sieved to give 326 g of an ellipsoidal calcium phosphate calcination product having a grain size of 580 to 1400 μm (sieve opening: 12 to 28 mesh). The yield of the granular calcium phosphate calcination product obtained per 340 g of the calcium phosphate powder packed in 10 pieces of the polyurethane foam was 96%. The relative density of the granular calcium phosphate calcination product was 98.3%, measured by the nitrogen adsorption method. A product of a high density was obtained.

On an electron microscope photograph (magnification: 26), 50 pieces of the granular calcium phosphate calcination product obtained were examined (10 pictures were taken …4 to 6 pieces of granular product per picture) and, D and A were measured. As a result, A/D was 0.76 and the number of granules having a shape such that the crossing point of A and D is within 0.3 D from both sides of the center of D was 92% based on the whole number of granules.

The granules were packed in a cavity formed on a jaw bone of a dog. The animal was sacrificed 30 days after the preparation of the specimen. The examination of the tissue in the specimen revealed, that the spaces of the calcium phosphate molding were completely filled with new bone material.

**Example 2**

A granular hydroxyl apatite calcination product was prepared in a manner similar to Example 1 except that hydroxyl apatite HCA-100S (Ca/P atomic ratio=1.67; grain diameter of the powder: 10 to 100 μm; specific surface area: 6 m$^2$/g) manufactured by Mitsui Toatsu Chemicals, Inc. was used instead of the calcium phosphate powder manufactured by Taihei Chemical Industrial Co., Ltd. 333 g ellipsoidal hydroxyl apatite calcination product having a grain size of 580 to 1400 μm (sieve opening: 12 to 28 mesh) was obtained based on 362 g of the hydroxyl apatite powder packed in the polyurethane foam and the yield was 92%. Further the relative density of the hydroxyl apatite calcination product was 94.5%.

In a manner similar to Example 1, D and A of the hydroxyl apatite calcination product were measured. As a result, A/D was 0.72 and the number of granules having a shape such that the crossing point of A and D is within 0.3 D from both sides of the center of D was 85% based on the whole number of granules.

**Example 3**

In 7 l of distilled water was dissolved 2500 g of commercially available calcium nitrate [Ca(NO$_3$)$_2$ · 4H$_2$O] and, 7.9 l of 28% ammonia water was slowly added to the solution, and further the obtained solution was diluted with 3 l of distilled water. On the other hand 840 g of commercially available ammonium hydrogen-phosphate [(NH$_4$)$_2$HPO$_4$] was dissolved in 10 l of distilled water. Further, 4.8 l of 28% ammonia water and 10 l of distilled water were supplemented to this solution. While keeping the former calcium nitrate aqueous solution at 20°C, the latter ammonium hydrogenphosphate aqueous solution (20°C) was dropwise added with stirring to react them. After completion of the dropwise addition, the mixture described above was heated 80°C and maintained for 20 minutes under reflux, while stirring was continued. After cooling, the reaction mixture was settled for further 2 days. Subsequently, the solution was dehydrated (2000 g) with a centrifugal dehydrater equipped with a polypropylene-made filter cloth having 16 μm (1000 mesh) pores and washed with distilled water until the system showed no alkaline property. The thus obtained calcium phosphate filter cake was dried and then ground into powder in a mortar. The powder was passed through a sieve of 105 μm (140 mesh) to give 920 g of calcium phosphate powder. The calcium phosphate powder was packed in 18 pieces of polyurethane foam "Everlightscott Filter" (HR-20; cell number: 17—25 cells/25 mm; porosity: 97%) manufactured by Bridgestone Corporation. The foam was put in a rubber form as in Example 1. Three pieces each in a group were pressed with a rubber press under 6 different pressures as shown in Table 1. They were put in an electric furnace and calcined for 2 hours at 1100°C, after elevating the temperature to 1100°C.

The obtained ellipsoidal calcium phosphate calcination moldings thus calcined were sieved to give ellipsoidal calcium phosphate calcination products having a grain size of 340 to 840 μm (sieve opening: 20 to 42 mesh) in yields shown in Table 1. The X ray diffraction pattern of the calcium phosphate calcination product was identical with that of hydroxyl apatite.

TABLE 1

| Pressure of rubber press (kg/cm²) | Granular calcium phosphate calcination product having a size of 340 to 840 µm | | Relative density (%) | A/D | E (%) note[3] |
|---|---|---|---|---|---|
| | (g) Note[1] | Yield (%) note[2] | | | |
| 250 | 74 | 72 | 86 | 0.71 | 83 |
| 350 | 91 | 88 | 93 | 0.70 | 88 |
| 1000 | 95 | 92 | 97 | 0.68 | 90 |
| 2000 | 98 | 95 | >99 | 0.67 | 91 |
| 5000 | 91 | 88 | >99 | 0.65 | 91 |
| 5500 | 80 | 78 | >99 | 0.65 | 93 |

Note[1] Total amount of 3 pieces of polyurethane foam.
Note[2] 103 g of calcium phosphate powder packed in 3 pieces of polyurethane foam.
Note[3] E indicates the ratio (%) of the number of granules having a shape such that the crossing point of A and D is within 0.3 D from both sides of the center of D to the whole number of granules.

Comparative Example 1

A calcium phosphate filter cake was prepared in a manner similar to Example 3. After drying, the calcium phosphate was broken by a hammer mill. The obtained broken calcium phosphate was calcined at the same temperatures as in Example 3.

The thus broken calcium phosphate calcination product was sieved to give 420 g of granular calcium phosphate calcination product having a grain size of 340 to 840 µm (sieve opening: 20 to 42 mesh). The yield of the obtained granular calcium phosphate calcination product was 40% based on the theoretical amount 1060 g of gelatin-like calcium phosphate precipitates. The relative density of the calcium phosphate calcination product was 99.2% but most of the granules had sharp edges. In a manner similar to Example 1, D and A of ghe granular calcium phosphate calcination product were measured. As a result, A/D was 0.52 and the number of granules having a shape such that the crossing point of A and D is within 0.3 D from both sides of the center of D was 52% based on the whole number of granules.

The granules were embedded in the raw bone of a dog in a manner similar to Example 1 and the tissue was observed. As a result, a large number of fibrous connective tissues were noted in the spaces of the calcium phosphate calcination product together with new bone.

Comparative Example 2

The calcium phosphate precipitates obtained in Example 3 by dehydrating with a centrifugal dehydrater and washing were dried at the same temperatures as in Example 1, as they were in a state of the filter cake. After drying, the calcium phosphate filter cake was put in a mortar having a diameter of 30 cm and ground so as to pass at least 95% of the dry calcium phosphate through a sieve of 2.0 mm (sieve opening: 8 mesh) the amount of the dry calcium phosphate having a maximum between 500 and 1000 µm (sieve opening: 16 and 32 mesh). The granular dry calcium phosphate obtained by grinding was put in an electric furnace and calcined at the same temperatures as in Example 1. The thus obtained calcium phosphate calcination product was again sieved to give 350 g of the granular calcium phosphate calcination molding having a grain size of 340 to 840 µm (sieve opening: 20 to 42 mesh; the same size as in Example 1). The yield of the obtained granular calcium phosphate calcination product was 33% based on the theoretical amount of 1060 g gelatin-like calcium phosphate precipitates. The remaining calcium phosphate calcination product mostly passed through a sieve of 250 µm (sieve opening: 60 mesh). The relative density of the calcium phosphate calcination product was 99.3% but most of the granules had sharp edges.

Example 4

A granular ellipsoidal calcium phosphate calcination product was prepared in a manner similar to Example 1 except that β-tricalcium phosphate powders (Ca/P atomic ratio=1.50; specific surface area: 1 m²/g) manufactured by Taihei Chemical Industrial Co., Ltd. was used. 319 g ellipsoidal calcium phosphate calcination product of having a grain size of 580 to 1400 µm (12 to 28 mesh) was obtained based on 355 g of

5

β-tricalcium phosphate packed in the polyurethane foam and the yield was 90%. Further the relative density of the calcium phosphate calcination product was 90.3%.

In a manner similar to Example 1, D and A were measured. As a result, A/D was 0.72 and the number of granules having a shape such that the crossing point of A and D is within 0.3 D from both sides of the center of D was 86% based on the whole number of granules.

Example 5

Ten pieces of pressed polyurethane foam packed with hydroxyl apatite powder were prepared in a manner similar to Example 1 except that hydroxyl apatite AN powder (Ca/P=1.67; specific surface area: 36 m²/g; grain diameter of the powder: 10 to 30 μm) manufactured by Central Glass Co., Ltd. was used. They were put in an electric furnace and calcined at 600°C for 3 hours. The thus obtained ellipsoidal hydroxyl apatite calcination product was sieved to give 295 g of the ellipsoidal hydroxyl apatite calcination product having a grain size of 580 to 1400 μm (12 to 28 mesh). The yield of the granular calcium phosphate calcination product was 89% based on 332 g of the hydroxyl apatite powder packed in the 10 pieces of polyurethane foam. Further the relative density of the calcium phosphate calcination product was 52% and, the hydroxyl apatite calcination product was a porous body in which the volume of the pores having a pore diameter of 70 to 250 Å was 83% based on the total pore volume.

In a manner similar to Example 1, D and A were measured. As a result, A/D was 0.75 and the number of granules having a shape such that the crossing point of A and D is within 0.3 D from both sides of the center of D was 82% based on the whole number of granules.

Example 6

Partially stabilized zirconia powder (specific surface area: 6 m²/g; Y₂O₃; 3 mol%) manufactured by Daiichi Kigenso Chemical Industry Co., Ltd. was packed in 10 pieces of polyurethane foam "Everlightscott Filter" (HR-13; cell number, 11—16 cells/25 mm; porosity, 97%) manufactured by Bridgestone Corporation. The foam was put in a rubber form (inner volume of 2 cm×5 cm×5 cm), and pressed with a rubber press under a pressure of about 2000 bar (2000 kg/cm²). Thereafter, the polyurethane foam containing the zirconia powder (the zirconia powder was in a granular form in the pores of the foam) was put in an electric furnace and calcined at 500°C for 2 to 3 hours. After elevating the temperature to 1500°C, calcination was performed for 2 hours. The thus obtained granular zirconia calcination product was sieved to give 570 g of the ellipsoidal zirconia calcination product having a grain size of 580 to 2400 μm (sieve opening: 8 to 28 mesh). The yield of the granular zirconia calcination product obtained was 95% based on 602 g of the zirconia powder packed in 10 pieces of the polyurethane foam. Further the relative density of the granular zirconia calcination product was 99% as measured by the nitrogen adsorption method and a product of high density was obtained.

In a manner similar to Example 1, D and A of the granular zirconia calcination product were measured. As a result, A/D was 0.68 and the number of granules having a shape such that the crossing point of A and D is within 0.3 D from both sides of the center of D was 82% based on the whole number of granules.

Example 7

A granular alumina calcination product was prepared in a manner similar to Example 1 except that alumina powder (specific surface area: 145 m²/g) manufactured by Catalysts & Chemicals Industries Co., Ltd. was used and the calcination was conducted at an electric furnace temperature of 1800°C. 279 g ellipsoidal alumina calcination product having a grain size of 580 to 1400 μm (12 to 28 mesh) was obtained based on 310 g of the alumina powder packed in the polyurethane foam and the yield was 90%. Further the relative density of the alumina calcination product was 96%.

In a manner similar to Example 1, D and A of the alumina calcination product were measured. As a result, A/D was 0.78 and the number of granules having a shape such that the crossing point of A and D is within 0.3 D from both sides of the center of D was 91% based on the whole number of granules.

Example 8

A granular alumina calcination product was prepared in a manner similar to Example 7 except that the calcination was conducted at an electric furnace temperature of 1200°C. The yield of obtained ellipsoidal alumina calcination product having a grain size of 720 to 2400 μm (sieve opening: 8 to 24 mesh) was 87%. Further the relative density of the alumina calcination product was 72% and, the alumina calcination product was a porous body in which the volume of the pores having a pore diameter of 1200 to 1600 Å was 81% based on the total pore volume.

In a manner similar to Example 1, D and A of the alumina calcination product were measured. As a result, A/D was 0.82 and the number of granules having a shape such that the crossing point of A and D is within 0.3 D from both side of the center of D was 84% based on the whole number of granules.

Example 9

An ellipsoidal calcium phosphate-zirconia mixture calcination product was prepared in a manner similar to Example 1 except that a mixture of hydroxyl apatite AN manufactured by Central Glass Co., Ltd. and partially stabilized zirconia powder manufactured by Daiichi Kigenso Chemical Industry Co., Ltd. (10:1

## EP 0 263 489 B1

in a weight ratio) was used. 285 g ellipsoidal calcium phosphate-partially stabilized zirconia mixture calcination product having a grain size of 580 to 1400 µm (12 to 28 mesh) was obtained based on 320 g of the mixed powder packed in the polyurethane foam and the yield was 39%. Further the relative density of the calcium phosphate-zirconia mixture calcination product was 93%.

In a manner similar to Example 1, D and A of the calcium phosphate-zirconia mixture calcination product were measured. As a result, A/D was 0.70 and the number of granules having a shape such that the crossing point of A and D is within 0.3 D from both sides of the center of D was 86% based on the whole number of granules.

## Claims

1. Granular inorganic moldings characterized in that the ratio A/D of the maximum diameter D and the maximum length A perpendicular to the maximum diameter of the granular molding is 0.5 to 0.9 and in that at least 80% of the granular moldings have a shape such that the crossing point of A and D is within 0.3 D from both sides of the center of D.

2. A granular inorganic molding as claimed in claim 1 wherein the relative density is at least 90%.

3. A granular inorganic molding as claimed in claim 1 which comprises a calcium phosphate as a main component.

4. A granular inorganic molding as claimed in claim 1 which comprises a metal oxide as a main component.

5. A process for producing granular inorganic moldings which comprises filling an inorganic powder capable of calcination into an organic porous body having a 3-dimensional net-like structure and subjecting it to press forming thereby to form granules of the inorganic powder in the pores of the porous body and then burning off said organic porous body and at the same time, calcining the granular moldings.

6. A process as claimed in claim 5 wherein said press forming is carried out as cold isostatic press fabrication.

7. A process as claimed in claim 5 wherein said calcination is performed at 500°C or higher.

8. The granular inorganic moldings of Claim 1 for use for reparing bone defects.

9. Use of the granular inorganic moldings of Claim 1 as a carrier for immobilized enzymes or catalysts.

## Patentansprüche

1. Körnige anorganische Formkörper, dadurch gekennzeichnet, daß das Verhältnis von A/D des größten Durchmessers D und der größten Länge A senkrecht zum größten Durchmesser des körnigen Formkörpers 0,5 bis 0,9 beträgt und daß mindestens 80% der körnigen Formkörper eine derartige Form aufweisen, daß der Kreuzungspunkt von A und D innerhalb 0,3 D von beiden Seiten der Mitte von D liegt.

2. Körniger anorganischer Formkörper nach Anspruch 1, wobei die relative Dichte mindestens 90% beträgt.

3. Körniger anorganischer Formkörper nach Anspruch 1, der ein Calciumphosphat als Hauptbestandteil umfaßt.

4. Körniger anorganischer Formkörper nach Anspruch 1, der ein Metalloxid als Hauptbestandteil umfaßt.

5. Verfahren zur Herstellung von körnigen anorganischen Formkörpern, das das Füllen eines anorganischen calcinierbaren Pulvers in einen organischen porösen Körper mit 3-dimensionaler netzartiger Struktur und dessen Formpressen, um dadurch Körner des anorganischen Pulvers in den Poren des porösen Körpers zu formen und das anschließende Wegbrennen des organischen porösen Körpers und gleichzeitig des Calcinieren der körnigen Formkörper umfaßt.

6. Verfahren nach Anspruch 5, wobei das Formpressen als kalte isostatische Preß-Herstellung ausgeführt wird.

7. Verfahren nach Anspruch 5, wobei das Calcinieren bei 500°C oder mehr durchgeführt wird.

8. Körnige anorganische Formkörper nach Anspruch 1 zur Verwendung zum Reparieren von Knochenschäden.

9. Verwendung der körnigen anorganischen Formkörper nach Anspruch 1 als Träger für gebundene Enzyme oder Katalysatoren.

## Revendications

1. Corps moulés non organiques, granulaires, caractérisés en ce que le rapport A/D du diamètre maximum D et de la longueur maximum A perpendiculaire au diamètre maximum du corps moulé granulaire est de 0,5 à 0,9 et en ce qu'au moins 80% des corps moulés granulaires ont une forme telle que le point d'intersection de A et D est à l'intérieur de 0,3 D à partir de chaque côté du centre de D.

2. Corps moulé non organique, granulaire tel que revendiqué à la revendication 1, dans lequel la densité relative est d'au moins 90%.

3. Corps moulé non organique, granulaire tel que revendiqué à la revendication 1, qui comprend un phosphate de calcium en tant que composant principal.

4. Corps moulé non organique, granulaire tel que revendiqué à la revendication 1, qui comprend un oxyde métallique en tant que composant principal.

5. Procédé pour produire des corps moulés non organiques, granulaires qui comprend l'introduction d'une poudre non organique apte à être calcinée dans un corps poreux organique présentant une structure à trois dimensions de type réseau et la soumission de celui-ci au formage par pressage, pour former ainsi des granules de la poudre non organique dans les pores du corps poreux puis le grillage dudit corps organique poreux et, en même temps, la calcination des corps moulés granulaires.

6. Procédé tel que revendique à la revendication 5, dans lequel ledit formage par pressage est effectué sous forme de fabrication par pressage isostatique à froid.

7. Procédé tel que revendiqué à la revendication 5, dans lequel ladite calcination est effectuée à 500°C ou plus.

8. Corps moulés non organiques, granulaires selon la revendication 1, pour l'utilisation pour réparer des défauts des os.

9. Utilisation des corps moulés non organiques, granulaires selon la revendication 1 comme support pour des enzymes ou des catalyseurs immobilisés.

1000 μm

Figure 1

Granule A type

Granule B type

Granule A type (in the range) --------X pieces
Granule B type (out of the range) ----Y pieces

X/X+Y > 80%

Figure 2

EP 0 263 489 B1